# EUROPEAN PATENT SPECIFICATION

(11) **EP 2 813 574 B1**
(45) Date of publication and mention of the grant of the patent: **20.02.2019**
(21) Application number: 13002972.1
(22) Date of filing: 10.06.2013
(51) Int. Cl.: C12N 15/86, C12N 7/00, A61K 39/155, C12N 5/10

(54) **Semi-live respiratory syncytial virus vaccine**
Halblebender respiratorischer Synzytialvirusimpfstoff
Vaccin contre le virus respiratoire syncytial semi-vivant

(43) Date of publication of application: 17.12.2014
(73) Proprietor: RSV Genius GmbH, 80333 München (DE)
(72) Inventor: Wiegand, Marian, D-81667 München (DE); Kaufmann, Christine, D-81479 München (DE)
(74) Representative: D Young & Co LLP

(56) References cited:
- EP-A1- 2 045 260
- WO-A2-01/92548
- ZIMMER G ET AL: "A Chimeric Respiratory Syncytial Virus Fusion Protein Functionally Replaces the F and HN Glycoproteins in Recombinant Sendai Virus", JOURNAL OF VIROLOGY, THE AMERICAN SOCIETY FOR MICROBIOLOGY, US, vol. 79, no. 16, 1 August 2005 (2005-08-01), pages 10467-10477, XP003013377, ISSN: 0022-538X, DOI: 10.1128/JVI.79.16.10467-10477.2005
- VOGES ET AL: "Recombinant Sendai virus induces T cell immunity against respiratory syncytial virus that is protective in the absence of antibodies", CELLULAR IMMUNOLOGY, ACADEMIC PRESS, SAN DIEGO, CA, US, vol. 247, no. 2, 1 June 2007 (2007-06-01), pages 85-94, XP022323568, ISSN: 0008-8749, DOI: 10.1016/J.CELLIMM.2007.07.005
- ZHAN ET AL: "Respiratory syncytial virus (RSV) fusion protein expressed by recombinant Sendai virus elicits B-cell and T-cell responses in cotton rats and confers protection against RSV subtypes A and B", VACCINE, ELSEVIER LTD, GB, vol. 25, no. 52, 5 November 2007 (2007-11-05), pages 8782-8793, XP022374863, ISSN: 0264-410X
- BART G JONES ET AL: "Sendai virus-based RSV vaccine protects African green monkeys from RSV infection", VACCINE, ELSEVIER LTD, GB, vol. 30, no. 5, 11 November 2011 (2011-11-11), pages 959-968, XP028436178, ISSN: 0264-410X, DOI: 10.1016/J.VACCINE.2011.11.046 [retrieved on 2011-11-19]
- STONE RAYCHEL ET AL: "Critical Role of the Fusion Protein Cytoplasmic Tail Sequence in Parainfluenza Virus Assembly", PLOS ONE, vol. 8, no. 4, April 2013 (2013-04), XP002707791, ISSN: 1932-6203

### SEQUENCE LISTING

<110> AmVac AG
<120> Semi-live respiratory syncytial virus vaccine
<130> 109817P485EP
<160> 3
<170> PatentIn version 3.5
<210> 1
   <211> 524
   <212> PRT
   <213> Artificial Sequence
<220>
   <223> Ectodomain of RSV strain ATCC VR-26 F protein
<400> 1
   Met Glu Leu Pro Ile Leu Lys Ala Asn Ala Ile Thr Thr Ile Leu Ala
   1               5                   10                  15
   Ala Val Thr Phe Cys Phe Ala Ser Ser Gln Asn Ile Thr Glu Glu Phe
               20                  25                  30
   Tyr Gln Ser Thr Cys Ser Ala Val Ser Lys Gly Tyr Leu Ser Ala Leu
           35                  40                  45
   Arg Thr Gly Trp Tyr Thr Ser Val Ile Thr Ile Glu Leu Ser Asn Ile
       50                  55                  60
   Lys Glu Asn Lys Cys Asn Gly Thr Asp Ala Lys Val Lys Leu Ile Asn
   65                  70                  75                  80
   Gln Glu Leu Asp Lys Tyr Lys Asn Ala Val Thr Glu Leu Gln Leu Leu
                   85                  90                  95
   Met Gln Ser Thr Thr Ala Ala Asn Asn Arg Ala Arg Arg Glu Leu Pro
               100                 105                 110
   Arg Phe Met Asn Tyr Thr Leu Asn Asn Thr Lys Lys Thr Asn Val Thr
           115                 120                 125
   Leu Ser Lys Lys Arg Lys Arg Arg Phe Leu Gly Phe Leu Leu Gly Val
       130                 135                 140
   Gly Ser Ala Ile Ala Ser Gly Ile Ala Val Ser Lys Val Leu His Leu
   145                 150                 155                 160
   Glu Gly Glu Val Asn Lys Ile Lys Ser Ala Leu Leu Ser Thr Asn Lys
                   165                 170                 175
   Ala Val Val Ser Leu Ser Asn Gly Val Ser Val Leu Thr Ser Lys Val
               180                 185                 190
   Leu Asp Leu Lys Asn Tyr Ile Asp Lys Gln Leu Leu Pro Ile Val Asn
   195 200 205
   Lys Gln Ser Cys Arg Ile Ser Asn Ile Glu Thr Val Ile Glu Phe Gln
   210 215 220
   Gln Lys Asn Asn Arg Leu Leu Glu Ile Thr Arg Glu Phe Ser Val Asn
   225 230 235 240
   Ala Gly Val Thr Thr Pro Val Ser Thr Tyr Met Leu Thr Asn Ser Glu
   245 250 255
   Leu Leu Ser Leu Ile Asn Asp Met Pro Ile Thr Asn Asp Gln Lys Lys
   260 265 270
   Leu Met Ser Asn Asn Val Gln Ile Val Arg Gln Gln Ser Tyr Ser Ile
   275 280 285
   Met Ser Ile Ile Lys Glu Glu Val Leu Ala Tyr Val Val Gln Leu Pro
   290 295 300
   Leu Tyr Gly Val Ile Asp Thr Pro Cys Trp Lys Leu His Thr Ser Pro
   305 310 315 320
   Leu Cys Thr Thr Asn Thr Lys Glu Gly Ser Asn Ile Cys Leu Thr Arg
   325 330 335
   Thr Asp Arg Gly Trp Tyr Cys Asp Asn Ala Gly Ser Val Ser Phe Phe
   340 345 350
   Pro Gln Ala Glu Thr Cys Lys Val Gln Ser Asn Arg Val Phe Cys Asp
   355 360 365
   Thr Met Asn Ser Leu Thr Leu Pro Ser Glu Val Asn Leu Cys Asn Val
   370 375 380
   Asp Ile Phe Asn Pro Lys Tyr Asp Cys Lys Ile Met Thr Ser Lys Thr
   385 390 395 400
   Asp Val Ser Ser Ser Val Ile Thr Ser Leu Gly Ala Ile Val Ser Cys
   405 410 415
   Tyr Gly Lys Thr Lys Cys Thr Ala Ser Asn Lys Asn Arg Gly Ile Ile
   420 425 430
   Lys Thr Phe Ser Asn Gly Cys Asp Tyr Val Ser Asn Lys Gly Val Asp
   435 440 445
   Thr Val Ser Val Gly Asn Thr Leu Tyr Tyr Val Asn Lys Gln Glu Gly
   450 455 460
   Lys Ser Leu Tyr Val Lys Gly Glu Pro Ile Ile Asn Phe Tyr Asp Pro
   465                 470                 475                 480
   Leu Val Phe Pro Ser Asp Glu Phe Asp Ala Ser Ile Ser Gln Val Asn
                   485                 490                 495
   Glu Lys Ile Asn Gln Ser Leu Ala Phe Ile Arg Lys Ser Asp Glu Leu
               500                 505                 510
   Leu His His Val Asn Ala Gly Lys Ser Thr Thr Asn
           515                 520
<210> 2
   <211> 24
   <212> PRT
   <213> Artificial Sequence
<220>
   <223> Transmembrane domain of SeV strain Fushimi (V52) F protein
<400> 2
   Thr Val Ile Thr Ile Ile Val Val Met Val Val Ile Leu Val Val Ile
   1               5                   10                  15
   Ile Val Ile Val Ile Val Leu Tyr
               20
<210> 3
   <211> 42
   <212> PRT
   <213> Artificial Sequence
<220>
   <223> Cytoplasmic domain of Sev strain Fushimi (v52) F protein
<400> 3
   Arg Leu Lys Arg Ser Met Leu Met Gly Asn Pro Asp Asp Arg Ile Pro
   1               5                   10                  15
   Arg Asp Thr Tyr Thr Leu Glu Pro Lys Ile Arg His Met Tyr Thr Asn
               20                  25                  30
   Gly Gly Phe Asp Ala Met Ala Glu Lys Arg
           35                  40

## Description

### FIELD OF THE INVENTION

The present invention relates to a semi-live respiratory syncytial virus (RSV) vaccine, which comprises a genome replication-deficient Sendai virus (SeV) vector expressing a chimeric RSV/SeV F protein. Furthermore, the present invention relates to a method for the production of the genome replication-deficient SeV vector of the present invention, and the use thereof in the treatment of RSV infections and RSV infection-related diseases.

### BACKGROUND OF THE INVENTION

Many of the viral vaccines used today, including those of measles and some influenza vaccines, are based on attenuated viruses and generate good and long-lasting prophylactic humoral and cellular immune responses (Amanna et al., N. Engl. J. Med. 357:1903-1915, 2007). Such live attenuated vaccines are created by reducing the virulence of the used virus, but still keeping it viable (or "alive").

However, safety of live vaccines is constantly being discussed as they have also been associated with genetic instability and residual virulence (Ehrenfeld et al., Expert. Rev. Vaccines 8:899-905, 2009). Possible reversion of attenuating mutations, as seen with the Sabin polio vaccine (Salk, D. and Salk, J., Vaccine 2:59-74, 1984; Kew et al., Annu. Rev. Microbiol. 59:587-635, 2005), or finding the right balance of attenuation, which complicates for instance the development of live attenuated respiratory syncytial virus (RSV) vaccines (Luongo et al., Vaccines 27:5667-5676, 2009), exemplify the shortcomings of live vaccines.

Given the limitations present in using live vaccines, viral vectors have emerged as potent and defined approaches with immunogenic characteristics similar to live attenuated vaccines (Abdulhaqq et al., Immunol. Res. 42:219-232, 2008; Liniger et al., Vaccine 27:3299-3305, 2009; Zhan et al., Vaccine 26:3480-3488, 2008; Slobod et al., Vaccine 22:3182-3186, 2004). However, live attenuated viral vectors often face similar safety concerns as the long-used live attenuated vaccines.

A group of viruses which has received significant attention from vaccine developers in the past is the group of non-segmented negative-strand RNA viruses (NNSV). These viruses have a very desirable safety profile since they contain an RNA genome and replicate only in the cytoplasm of host cells, excluding any possibility of integration into the host genome to cause insertional mutagenesis. Moreover, recombination events have not yet been observed (Bukreyev et al., J. Virol. 80:10293-10306, 2006). The NNSV comprise four families, of which members of the *Rhabdoviridae* (e.g., vesicular stomatitis virus (VSV) and rabies virus (RV)) and the *Paramyxoviridae* (e.g., Sendai virus (SeV) and human parainfluenza virus (hPIV)) have been preferentially used for the development of candidate viral vector vaccines (Schmidt et al., J. Virol. 75:4594-4603, 2001; Bukreyev et al., J. Virol. 80:10293-10306, 2006).

Using NNSV as vaccine backbones, various viral vaccine vector candidates have been developed. For example, a hPIV2/hPIV3 viral vaccine vector was produced by incorporation of HN and F proteins of human parainfluenza virus type 2 (hPIV2) having their cytoplasmic domains replaced with the corresponding ones of human parainfluenza virus type 3 (hPIV3) into a viral vector based on hPIV3 (Tao et al., J. Virol. 74:6448-6458, 2000). In addition, a bovine/human attenuated PIV3 vaccine vector was described, which expresses the F protein of hPIV3 in a bovine PIV3 (bPIV3) backbone (Haller et al., J. Virol. 74:11626-35, 2000). Further known is a bovine PIV3-based vaccine candidate expressing the F and NH proteins of human PIV3 and the full-length, native F protein of human RSV, which was found to confer protection from RSV infection in African green monkeys (Tang et al., J. Virol. 79:11198-11207, 2004).

Another candidate viral vector vaccine known in the art is based on a genome replication-deficient Sendai virus (SeV) (Wiegand et al., J. Virol. 81:13835-13844, 2007; WO 2006/084746 A1). This vector is still capable of expressing genes *in vitro,* as recently shown (Bossow et al., Open Virol. J. 6:73-81, 2012). *In vivo* safety of the replication-deficient SeV-based viral vaccine vector, however, concerning its replication-deficient nature and genetic stability, has still to be proven. In addition, the *in vitro* gene expression is, due to its replication-deficiency, reduced compared to that of replication-competent Sendai vectors (Bossow et al., Open Virol. J. 6:73-81, 2012). Therefore, it is a challenging task to recombinantly engineer a replication-deficient Sendai vector that efficiently expresses and displays selected immunogenic peptides or proteins to the immune system in a manner that results in the desired efficient humoral and/or cellular immune responses *in vivo.*

A well-known, but difficult to treat, pathogenic virus is the respiratory syncytial virus (RSV). RSV is a leading cause of serious respiratory diseases in young children and the elderly worldwide (Collins P.L. and Crowe J.E. Jr, Respiratory syncytial virus and metapneumovirus, in: Fields Virology, Eds. Knipe D.M. and Howley P., Philadelphia: Lippincott-Williams and Wilkins, Wolters Kluwer Business, 2007:1601-1646). RSV is also a major pathogen in chronic obstructive pulmonary disease (COPD) patients (Hacking, D. and Hull, J., J. Infect. 45:18-24, 2002). However, despite the significant RSV vaccine development efforts in recent times, there is still no vaccine available today against this pathogen.

Thus, there remains an urgent need for a safe RSV vaccine that is effective in the treatment of patients, in particular children and the elderly, suffering from RSV infections and RSV infection-related diseases.

### SUMMARY OF THE INVENTION

The invention is as claimed in claims 1-14. The present invention fulfills the need presented above by providing a genome replication-deficient Sendai virus (SeV) vector expressing a chimeric RSV/SeV F (fusion) protein (in the following referred to as "genome replication-deficient SeV vector of the present invention" or "rdSeV vector of the present invention"). The rdSeV vector of the present invention can be efficiently produced in high amounts and elicits strong humoral and cellular immune responses against RSV while at the same time being safe. It is therefore well-suited for use as a "semi-live" RSV vaccine, i.e. a vaccine that is exceptionally effective (like "live vaccines") and yet particularly safe (like "dead vaccines").

In a first aspect, the present invention provides a genome replication-deficient Sendai virus (SeV) vector comprising a nucleic acid that is modified in the phosphoprotein (P) gene to encode a mutant P protein lacking amino acids 2-77, wherein the nucleic acid further encodes a chimeric F protein comprising a respiratory syncytial virus (RSV) F ectodomain, a SeV F transmembrane domain, and lacks a cytoplasmic domain.

In another aspect, the present invention provides a host cell comprising a genome replication-deficient Sendai virus (SeV) vector of the present invention, the nucleic acid of the genome replication-deficient SeV vector of the present invention or a complement thereof, and/or a DNA molecule encoding the nucleic acid of the genome replication-deficient SeV vector of the present invention or encoding a complement of the nucleic acid.

In a further aspect of the present invention, there is provided a method for producing the genome replication-deficient Sendai virus (SeV) vector of the present invention, comprising (i) culturing a host cell of the present invention, and (ii) collecting the genome replication-deficient SeV vector from the cell culture.

According to another aspect, the present invention provides a vaccine comprising the genome replication-deficient Sendai virus (SeV) vector of the present invention and one or more pharmaceutically acceptable carriers.

In yet another aspect, the present invention relates to the use of a genome replication-deficient Sendai virus (SeV) vector of the present invention in the treatment of RSV infections or RSV infection-related diseases in a mammal, particularly in a human subject, more particularly in a human infant or child, an elderly human, a human immunocompromised individual, a transplant recipient, or an individual suffering from a chronic disease.

Preferred embodiments of the present invention are set forth in the appended dependent claims.

### BRIEF DESCRIPTION OF THE DRAWINGS

The foregoing summary, as well as the following detailed description and examples, will be better understood when read in conjunction with the appended drawings.

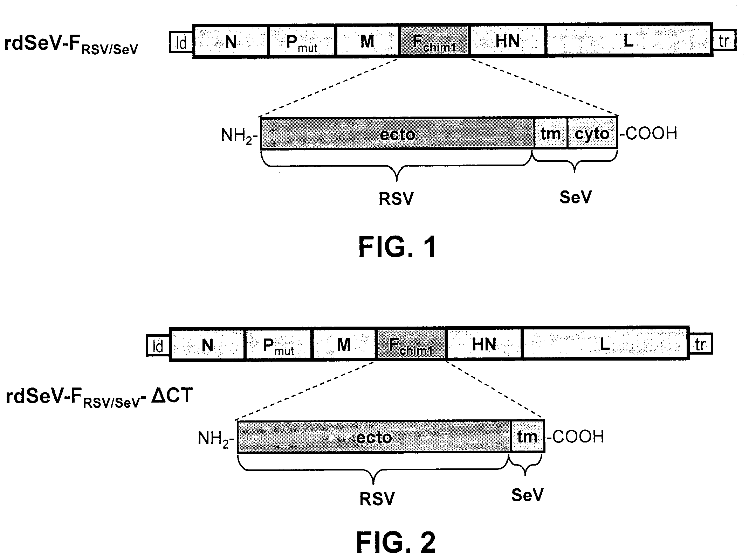

FIG. 1 is a schematic representation showing the genome structure of the genome replication-deficient SeV vector of the present disclosure expressing a chimeric RSV/SeV F protein, designated as "rdSeV-F_{RSV/SeV}" vector. The ectodomain of SeV F was replaced by its RSV-derived counterpart resulting in the following chimeric F ("F_{chim1}") protein: RSV ectodomain ("ecto"; amino acids 1-524 of RSV F), SeV transmembrane domain ("tm"; amino acids 500-523 of SeV F), and cytoplasmic domain ("cyto"; amino acids 524-565 of SeV F). In the "Pₘᵤₜ" ORF, the first 76 amino acids were deleted (PΔ2-77) to obtain a replication-deficient vaccine vector.
FIG. 2 is a schematic representation showing the genome structure of a variant of the genome replication-deficient SeV vector of the present invention, designated as "rdSeV-F_{RSV/SeV}-ACT. This variant is identical to the rdSeV-F_{RSV/SeV} shown in FIG. 1 but lacks the entire cytoplasmic domain except for the N-terminal first two amino acids (amino acids 524-525 of SeV F). In the "Pₘᵤₜ" ORF, the first 76 amino acids were deleted (PΔ2-77) to obtain a replication-deficient vaccine vector.

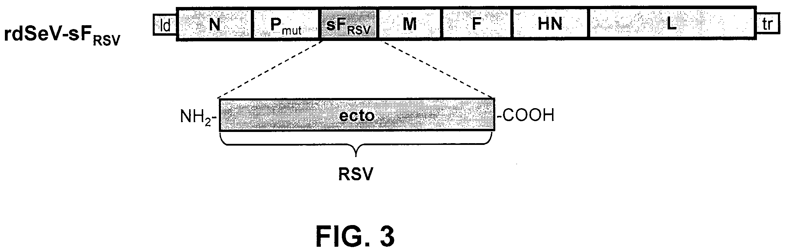

FIG. 3 is a schematic representation showing the genome structure of a comparative genome replication-deficient SeV vector, designated "rdSeV-sF_{RSV}, which expresses a soluble F (sF) protein of RSV. The ORF of the RSV F ectodomain (amino acids 1-524 of RSV F) was inserted as an additional transcription unit ("sF_{RSV}") downstream of the P gene. In the "Pₘᵤₜ" ORF, the first 76 amino acids were deleted (PΔ2-77) to obtain a replication-deficient vaccine vector.

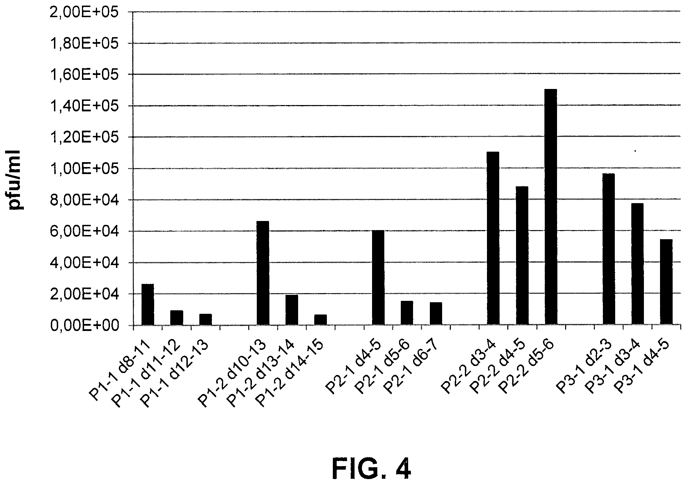

FIG. 4 is a bar graph showing the production efficiency of the genome replication-deficient SeV vector of the present disclosure (rdSeV-F_{RSV/SeV}). The rdSeV-F_{RSV/SeV} vector was produced in VPN cells stably transfected with expression plasmids containing the genes coding for SeV P and N proteins. Different production runs of both vectors at different passaging levels ("P") were performed in comparison (P1-1, P1-2, P2-1, P2-2, P3-1), and samples from the cell culture supernatants were taken at different time points during production, e.g. at day 8-11 ("d8-11"), day 11-12 ("d11-12"), and so forth. The vector titers (pfu/ml) of the samples taken were then determined.

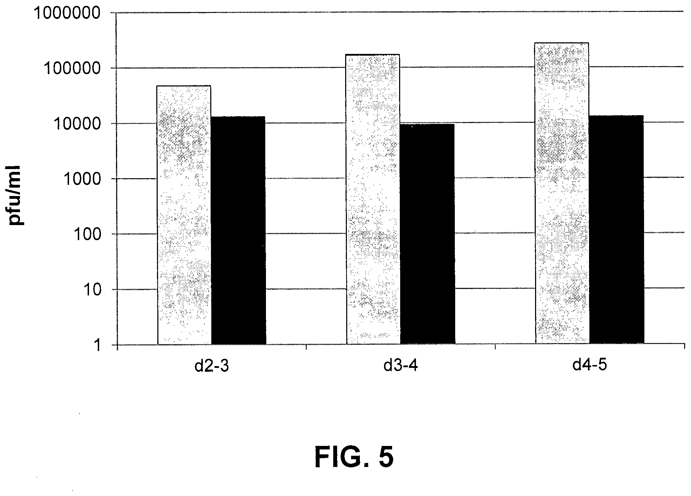

FIG. 5 is a bar graph showing the production efficiency for rdSeV-F_{RSV/SeV}(black bars) and a variant thereof which lacks the entire cytoplasmic domain except for the N-terminal first two amino acids (designated as "rdSeV-F_{RSV/SeV}-ΔCT") (white bars). The vector titers of cell culture supernatants in pfu/ml were determined at day 3 ("d2-3"), day 4 ("d3-4"), and day 5 ("d4-5").

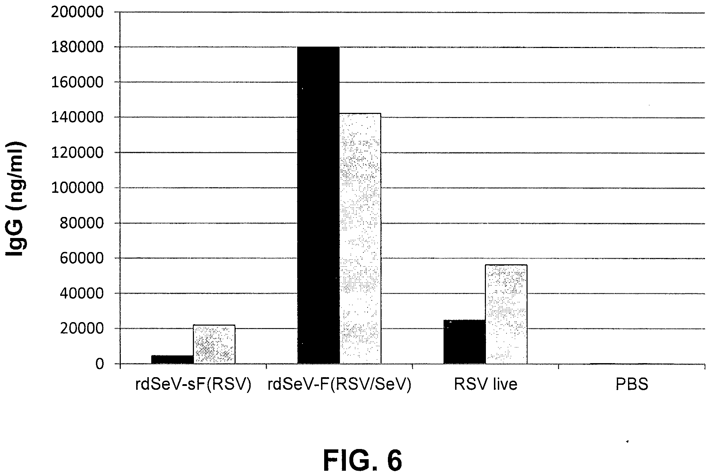

FIG. 6 is a bar graph showing the RSV-specific serum IgG levels following intranasal (i.n.) administration (black bars) and intramuscular (i.m.) administration (white bars) for rdSeV-sF_{RSV}, rdSeV-F_{RSV/SeV}, RSV live, and PBS. Serum samples were drawn 14 days after the last immunization and assayed for the presence of RSV-specific IgG antibodies by ELISA.

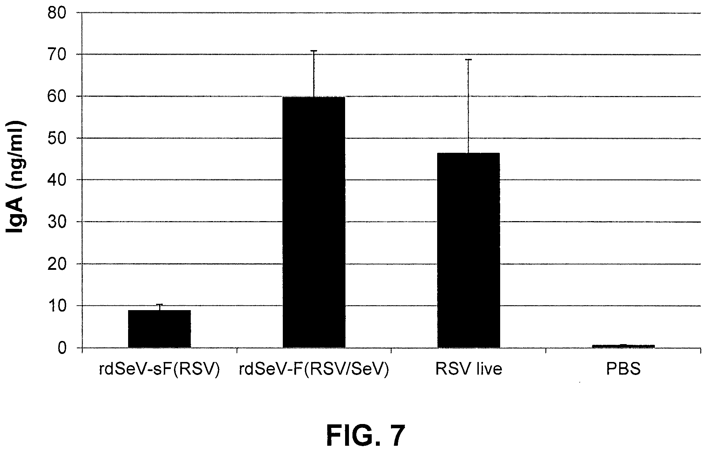

FIG. 7 is a bar graph showing the RSV-specific IgA (mucosal antibodies) response (ng/ml) in nasal washes (NW) of mice inoculated with rdSeV-sF_{RSV}, rdSeV-F_{RSV/SeV}, RSV live and PBS. The NW samples were collected 14 days after the last immunization and assayed for the presence of RSV-specific IgA antibodies by ELISA.

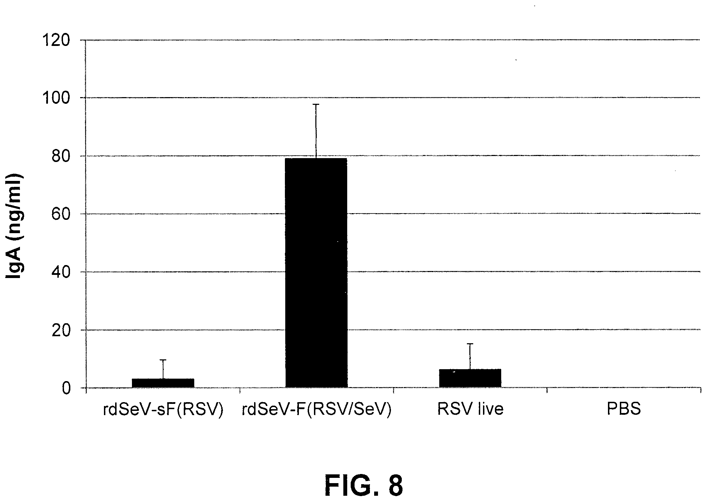

FIG. 8 is a bar graph showing the RSV-specific IgA (mucosal antibodies) response (ng/ml) in bronchoalveolar lavages (BAL) of mice inoculated with rdSeV-sF_{RSV}, rdSeV-F_{RSV/SeV}, RSV live and PBS. The BAL samples were collected 14 days after the last immunization and assayed for the presence of RSV-specific IgA antibodies by ELISA.

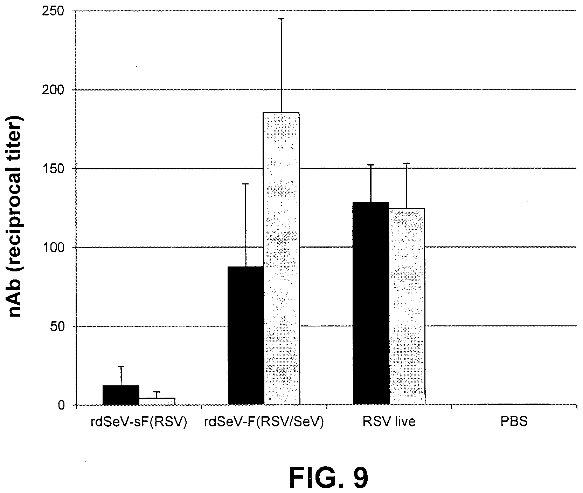

FIG. 9 is a bar graph showing the neutralizing antibody titers in serum following intranasal (i.n.) (black bars) and intramuscular (i.m.) (white bars) infection of mice with rdSeV-sF_{RSV}, rdSeV-F_{RSV/SeV}, RSV live and PBS. Serum samples were drawn 14 days after the last immunization and assayed for the presence of RSV-specific IgG antibodies by ELISA.

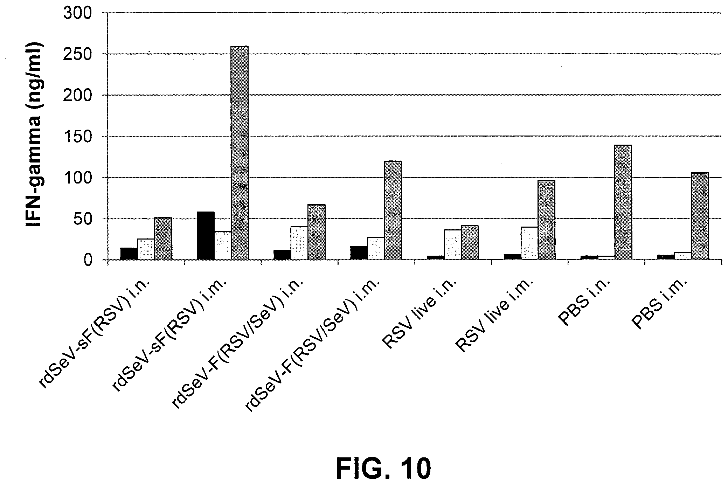

FIG. 10 is a bar graph showing the IFN-gamma expression (ng/ml) of re-stimulated splenocytes after intranasal (i.n.) and intramuscular (i.m.) inoculation with rdSeV-sF_{RSV}, rdSeV-F_{RSV/SeV}, RSV live and PBS. Splenocytes were re-stimulated either with inactivated RSV (white bars) or with Concanavalin A (grey bars) as positive controls. As negative control, there were used unstimulated splenocytes (black bars).

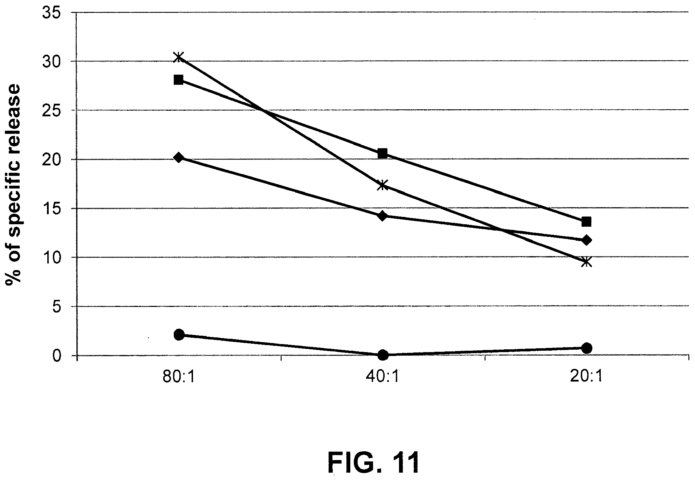

FIG. 11 is a diagram showing the lytic activity (CTL response) of mice RSV-specific cytotoxic T lymphocytes (CTL) stimulated by intranasal application of rdSeV-sF_{RSV} (◆), rdSeV-F_{RSV/SeV} (■), RSV live (x) and PBS (●). The cytotoxic activity (% of specific release) was measured using RSV infected P815 cells (MOI 0.1). Splenocytes were added to the P815 target cells at ratios of 80:1, 40:1 and 20:1.

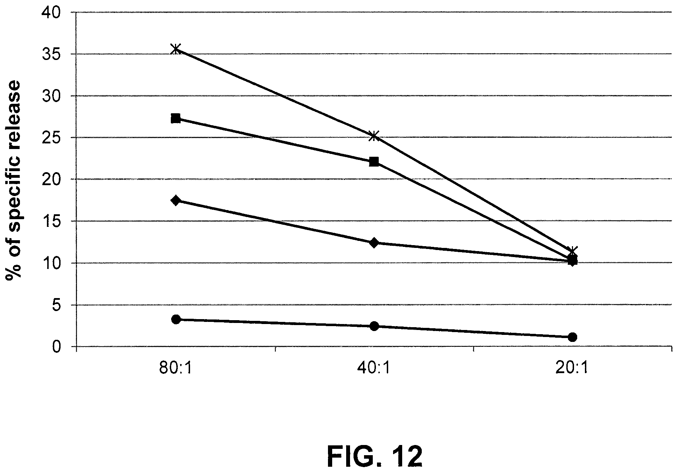

FIG. 12 is a diagram showing the lytic activity (CTL response) of mice RSV-specific cytotoxic T lymphocytes (CTL) stimulated by intramuscular application of rdSeV-sF_{RSV} (◆), rdSeV-F_{RSV/SeV} (■), RSV live (x) and PBS (●). The cytotoxic activity (% of specific release) was measured using RSV infected P815 cells (MOI 0.1).

### DETAILED DESCRIPTION OF THE INVENTION

In accordance with the present invention, the genome replication-deficient SeV vector of the present invention does not only provide a superior safety profile, but also unexpectedly exhibits the ability to stimulate the immune system in a very efficient manner to confer effective protection against RSV infections. In particular, it was found that the genome replication-deficient SeV vector of the present invention can be conveniently administrated via the intranasal route to highly efficiently induce local IgA antibodies, which play a critical role in mucosal immunity, and systemic IgG antibodies, including neutralizing antibodies, as well as cell-mediated protective immune responses.

Another surprising finding of the present invention is that the genome replication-deficient SeV vector of the present invention can be highly efficiently produced in large amounts using cells that are qualified for human use. This allows for the cost-efficient production of the viral vaccine vector of the present invention, which is of utmost importance for a commercial vaccine. Furthermore, the genome replication-deficient SeV vector of the present invention can be produced in a simple and reproducible way and, due its small genome size, allows for constant and reliable sequence surveillance.

In a first aspect, the present invention provides a genome replication-deficient Sendai virus (SeV) vector. This vector comprises a nucleic acid that is modified in the phosphoprotein (P) gene to encode a mutant P protein lacking amino acids 2-77. The nucleic acid further encodes a specific chimeric RSV/SeV F protein. As used herein, a "Sendai virus vector" or "SeV vector" is an infectious virus comprising a viral genome. This is, the recombinant rdSeV vector of the present invention can be used for the infection of cells and cell lines, in particular for the infection of living animals including humans to induce immune responses against RSV infections.

Within the context of the present invention, the term "nucleic acid" is used in the broadest sense and encompasses single-stranded (ss) DNA, double-stranded (ds) DNA, cDNA, (-)-RNA, (+)-RNA, dsRNA and the like. However, when the nucleic acid is part of and included in the rdSeV vector of the present invention, the nucleic acid is negative-strand RNA ((-)-ssRNA). In this case, the nucleic acid corresponds typically to the genome of the rdSeV of the present invention. Further, the term "encoding", as used herein, refers to the inherent property of a nucleic acid to serve as a template for the synthesis of another nucleic acid (e.g., mRNA, negative-strand RNA ((-)-ssRNA) or positive-strand RNA ((+)-ssRNA) and/or for the synthesis of oligo- or polypeptides ("proteins"). This is, a protein is "encoded" if transcription and translation results in the production of the protein in a cell or other biological system.

The SeV which serves as the backbone of the genome replication-deficient SeV vector of the present invention may be any known SeV strain. Suitable examples include, but are not limited to, the Sendai Fushimi strain (ATCC VR105), the Sendai Harris strain, the Sendai Cantell strain or the Sendai Z strain. The rdSeV of the present invention is further characterized by being replication-deficient (replication-defective). This is achieved by modifying the SeV backbone in the phosphoprotein (P) gene to delete the N-terminal 76 amino acids (PΔ2-77 of the P protein), as described previously (Bossow et al., Open Virol. J. 6:73-81, 2012; WO 2006/084746 A1). The resulting SeV/PΔ2-77 vector is replication-deficient, i.e. unable to synthesize new genomic templates in non-helper cell lines, but still transcription-competent, i.e. capable of primary transcription and gene expression, as shown previously (Bossow et al., Open Virol. J. 6:73-81, 2012).

Without being bound to any particular theory, it is believed that the deletion in the P protein, an essential component of the viral RNA-dependent RNA polymerase (vRdRp) carrying out both viral transcription and viral replication, uncouples the replication and transcription activities of the vRdRp. While this leads to a complete loss of the replication ability, the SeV/PΔ2-77 vector is still able to carry out primary transcription, including both early and late primary transcription. "Early primary" transcription refers to the first transcriptional events in an infected host cell, where the viral RNA genome is transcribed by the vRdRp molecules that were originally included in the SeV viral particles. "Late primary transcription" refers to the phase in which *de novo* protein synthesis begins and transcription is increasingly carried out by newly synthesised vRdRp.

Disclosed herein, the chimeric RSV/SeV protein encoded by the nucleic acid of the rdSeV vector of the present disclosure comprises (i) an ectodomain of the respiratory syncytial virus (RSV) F protein, or an immunogenic fragment or mutant thereof, (ii) a transmembrane domain of a SeV F protein, or a functional fragment or mutant thereof, and, optionally, (iii) a cytoplasmic domain of a SeV F protein, or any fragment or mutant thereof.

The term "comprise", as used herein, is intended to encompass both the open-ended term "include" and the closed term "consist (of)". Thus, the nucleic acid of the rdSeV vector of the present invention may further encode other heterologous proteins or chimeric proteins resulting in, for example, a bivalent viral vector vaccine (e.g., directed against RSV and hPIV).

Within the present invention, the above-mentioned transmembrane domain of SeV may correspond to amino acids 500-523 of a SeV F protein. The RSV ectodomain may correspond to amino acids 1-524 of a RSV F protein. Thus, the chimeric RSV/SeV F protein may comprise 590 amino acids, of which amino acids 1-524 define the RSV ectodomain, and amino acids 525-548 define the SeV transmembrane domain. Deletion variants and mutants of this 590 amino acid chimeric RSV/SeV F protein are also within the scope of the present disclosure, wherein the "fragments" and "mutants" of the ectodomain, the transmembrane domain and the cytoplasmic domain are as defined below.

Preferably, the RSV ectodomain has the amino acid sequence shown in SEQ ID NO: 1 (ectodomain of RSV strain ATCC VR-26 (Long strain) F protein; GenBank accession no. AY911262, Translation AAX23994), or is an immunogenic fragment or mutant thereof. Preferably, the SeV transmembrane domain has the amino acid sequence shown in SEQ ID NO: 2 (transmembrane domain of SeV strain Fushimi F protein; GenBank accession no. U06432, Translation AAC54271), or is a functional fragment or mutant thereof. Preferably, the SeV cytoplasmic domain has the amino acid sequences shown in SEQ ID NO: 3 (cytoplasmic domain of SeV strain Fushimi F protein; GenBank accession no. U06432, Translation AAC54271), or is any fragment or mutant thereof.

It is also preferred that the RSV ectodomain, the SeV transmembrane domain, and the SeV cytoplasmic domain are as defined above, except that the amino acid sequence of the RSV ectodomain shown in SEQ ID NO: 1 contains one or more, preferably all, point mutations selected from the group consisting of Glu66Gly, Val76GIu, Asn80Lys, Thr101Ser and Ser211Asn, and/or the amino acid sequence of the SeV cytoplasmic domain shown in SEQ ID NO: 3 contains the single point mutation Gly34Arg. Particularly preferred, the chimeric RSV/SeV F protein has an amino acid sequence as defined by SEQ ID NOs: 1-3, or an amino acid sequence as defined by SEQ ID NOs: 1-3 containing all six point mutations indicated above.

In the context of the present disclosure, the term "fragment" refers to a part of a polypeptide or protein domain generated by an amino-terminal and/or carboxy-terminal deletion. Preferably, the amino-terminal- and/or carboxy-terminal deletion is no longer than 10 or 5 amino acids, particularly 1, 2 or 3 amino acids. The term "immunogenic", as used herein, means a fragment or mutant of the RSV ectodomain that is still capable of eliciting a humoral and/or cellular immune response. Preferably, the immunogenic fragment or mutant, upon fusing it to the transmembrane domain having the amino acid sequence of SEQ ID NO: 2 and the cytoplasmic domain having the amino acid sequence of SEQ ID NO: 3, elicits a humoral and/or cellular immune response to a degree equal to or higher than 10%, 20%, 40%, 60% or 80% of that achieved by the full-length chimeric RSV/SeV F protein defined by the amino acid sequences of SEQ ID NOs: 1-3. The term "functional", as used herein, refers to a transmembrane domain fragment or mutant that is functionally equivalent to the transmembrane domain, i.e. a fragment or mutant which is still capable of anchoring the chimeric RSV/SeV F protein to the membrane.

Within the present invention, the SeV cytoplasmic domain (sometimes also referred to as "cytoplasmic tail") is as short as one amino acid or two to five amino acids. In this case, the respective chimeric RSV/SeV F protein may be referred to as "essentially lacking" a cytoplasmic domain. The term "essentially lacking", as used herein, also refers to chimeric RSV/SeV F proteins which entirely lack a SeV cytoplasmic domain. As demonstrated in the examples below, a variant of the chimeric RSV/SeV F protein that lacks the entire SeV cytoplasmic domain, except for the first and second N-terminal amino acids (e.g., amino acids 1 and 2 of SEQ ID NO: 3), was unexpectedly found to allow for a very high production efficiency, even higher than that achieved with the RSV/SeV F protein with the full-length SeV cytoplasmic domain. Therefore, since the cytoplasmic domain appears to be dispensable, chimeric RSV/SeV F proteins essentially lacking the cytoplasmic domain are encompassed by the present invention.

The term "mutant", as used herein, refers to a mutated polypeptide or protein domain, wherein the mutation is not restricted to a particular type of mutation. In particular, the mutation includes single-amino acid substitutions, deletions of one or multiple amino acids, including N-terminal, C-terminal and internal deletions, and insertions of one or multiple amino acids, including N-terminal, C-terminal and internal insertions, and combinations thereof. The number of inserted and/or deleted amino acids may be 1 to 10, particularly 1 to 5. In addition, 1 to 20, particularly 1 to 10, more particularly 1 to 5 amino acids may be mutated to (substituted by) another amino acid. Furthermore, the term "mutant" may also encompass mutated ectodomains, mutated transmembrane domains and mutated cytoplasmic domains, which are at least 75%, preferably at least 85%, more preferably at least 95%, and most preferably at least 97% identical to the amino acid sequence shown in SEQ ID NO: 1 (ectodomain of RSV strain ATCC VR-26 (Long strain) F protein), SEQ ID NO: 2 (transmembrane domain of SeV strain Fushimi F protein), and SEQ ID NO: 3 (cytoplasmic domain of SeV strain Fushimi F protein), respectively.

The SeV used as backbone and the SeV from which the transmembrane and cytoplasmic domains are derived may be the same or different. However, since the rdSeV of the present invention is generally constructed by replacing the SeV F ectodomain of the SeV backbone with the corresponding RSV F ectodomain, the SeV portion of the chimeric F protein is typically derived from the SeV that is used as backbone of the rdSeV vector of the present invention.

Suitable SeV strains for use as backbone and/or for construction of the chimeric RSV/SeV F protein include the Sendai Fushimi strain (ATCC VR-105), the Sendai Harris strain, the Sendai Cantell strain and the Sendai Z strain. Likewise, the RSV ectodomain may be derived from a RSV F protein from any recombinant or naturally-occurring RSV strain, preferable from a human SeV strain, such as A2, long, or B strains.

In one embodiment of the present invention, the nucleic acid of the genome replication-deficient SeV vector of the present invention encodes a soluble RSV F protein in addition to the chimeric RSV/SeV F protein. A "soluble F protein" within the meaning of the present invention is an F protein that lacks any stretch of amino acids which locates the F protein to the membrane and, in particular, refers to an F protein lacking both the transmembrane domain and the cytoplasmic domain. Thus, the soluble RSV F protein may be the ectodomain of a RSV F protein. The terms "fragment", "immunogenic", and "mutant" have the same meaning as defined above.

In a preferred embodiment, the soluble RSV F protein corresponds to amino acids 1-524 of a RSV F protein. In a particularly preferred embodiment, the soluble RSV F protein is the ectodomain of the RSV ATCC VR-26 strain (Long strain) F protein having the sequence shown in SEQ ID NO: 1.

If high expression of the heterologous gene encoding the soluble RSV F protein (in the following referred to as "sF transgene") is desired, the sequence is preferably inserted into the 3' region of the viral negative-strand RNA genome. The reason is that negative-strand RNA viruses like SeV most efficiently transcribe transcription units at the 3' end of their negative-strand RNA genome. Transcript levels of genes further downstream gradually decrease, which is a phenomenon known as transcriptional gradient. This allows regulating the expression level of a heterologous transgene by inserting it at different sites in the viral genome. Within the present invention, it is preferred that the sF transgene is inserted between the P (i.e. Pₘᵤₜ; PΔ2-77) gene and the M gene.

The sF transgene may be inserted as a transcriptional cassette, comprising the nucleic acid sequence encoding the soluble RSV F protein operatively linked to a transcription start sequence, a transcriptional terminator and, preferably, translation signals. The sF transgene may also be operatively linked with an mRNA stabilizing element. For instance, a Woodchuck hepatitis virus post-trancriptional regulatory element (WPRE) may be inserted into the 3'UTR and/or 5'UTR region of the sF transgene in order to stabilize its mRNA and prolong its expression.

The incorporation of the sF transgene encoding a soluble RSV F protein allows for the presentation of RSV antigens in two different ways, namely as a chimeric RSV/SeV F surface protein displaying the RSV antigen as structural vector component being embedded in the viral envelope, and as a soluble RSV F protein. Thus, the additional expression of a soluble RSV F protein may assist in inducing a more effective and broad immune response involving the humoral and cellular arms of the immune system.

In another embodiment of the present invention, the nucleic acid of the rdSeV vector of the present invention does not encode a soluble RSV F protein, or any fragment or mutant thereof. Furthermore, within the context of the present invention, it is preferred that the rdSeV vector of the present invention does not encode a chimeric F protein, other than the chimeric RSV/SeV F protein, described in detail herein and, preferably, does also not encode a soluble RSV F protein. Also, the chimeric RSV/SeV F protein described in detail herein is preferably the sole heterologous protein expressed by the rdSeV of the present invention.

In addition to the modifications described above, the SeV vector of the present invention may include other modifications. In particular, it may be modified to carry additional mutations in one or more viral genes. For example, the rdSeV vector of the present invention may additionally contain one or more mutations in at least one of the genes coding for viral envelope proteins. These mutations can be introduced by recombinant techniques as known in the art and may lead to different effects, such as altered viral cell specificity.

The rdSeV vector of the present invention may also have one or more mutation in the C, W, and/or V open reading frames (ORFs) as a result of N-terminal deletions in the viral P protein, because the C, W, and V ORFs overlap with the N-terminal ORF of the P gene. Furthermore, the rdSeV vector of the present invention may additionally have a deletion of the alternative start codon ACG of the C' gene. The C' gene encodes a non-structural protein known to exhibit an anti-IFN response activity in infected cells. The deletion of the start codon of the C' gene was found to result in increased expression levels of heterologous gene products in infected target cells.

In a second aspect, the present invention provides a host cell, which comprises a genome replication-deficient Sendai virus (SeV) vector of the present invention, a nucleic acid of the genome replication-deficient SeV vector of the present invention or a complement thereof, and/or a DNA molecule encoding the nucleic acid of the genome replication-deficient SeV vector of the present invention or encoding a complement of the nucleic acid.

A "complement" within the meaning of the present invention means a nucleotide sequence which is complementary to the sequence of the nucleic acid (i.e. an "antisense" nucleic acid). In this regard, it is noted that the nucleic acid generally corresponds to the genome of the rdSeV of the present invention. This is, the complement of the nucleic acid generally corresponds to the antigenome of the rdSeV of the present invention.

The host cell may be either a rescue cell (or "virus generating cell") or a helper cell (or "amplification cell"). The rescue cell is used for the initial production of the rdSeV vector of the present invention. The rescue cell is typically a eukaryotic cell, particularly a mammalian cell, which usually expresses a heterologous DNA-dependent and/or RNA-dependent RNA polymerase, such as T7 RNA polymerase or the homologous cellular RNA polymerase II. The gene encoding the heterologous DNA-dependent RNA polymerase may be integrated into the rescue cell's genome or present in an expression plasmid.

The rescue cell must further express a functional SeV P protein as well as SeV N and L proteins so that the rdSeV vector of the present invention can be assembled. The expression of these viral proteins is typically achieved by transfecting the rescue cell with one or more expression plasmids carrying the respective P, N and L genes. A suitable rescue cell for use herein is a BSR-T7 cell, which contains the gene for the T7 RNA polymerase stably integrated in its genome, and which has been transfected with expression plasmids harbouring the genes for the SeV P, N and L proteins (Buchholz et al., J. Virol. 73:252-259, 1999).

In order to initially produce the rdSeV vector of the present invention, a DNA molecule encoding the nucleic acid of the rdSeV of the present invention or its antisense nucleic acid is transfected into a rescue cell. The cell transfection can be carried out in accordance with procedures known in the art, for example chemically with FuGENE 6 or FuGENE HD (Roche) reagents as described by the manufacturer, or by electroporation. The transfected DNA molecule is typically a plasmid carrying the cDNA of the nucleic acid of the rdSeV of the present invention. Since the DNA molecule is usually transcribed by a heterologous DNA-dependent RNA polymerase of the rescue cell, the DNA molecule preferably further includes a transcriptional signal, e.g. a T7 promoter, and a terminator sequence operatively linked with the viral genomic sequence. It may further include a ribozyme sequence at its 3' end, which allows for cleavage of the transcript at the 3' end of the viral sequence. The DNA molecule is further preferably suitable for propagation in a prokaryotic helper cell (e.g., *Escherichia coli*) and/or in a eukaryotic helper cell, in particular in a mammalian helper cell. After packaging the recombinant viral genome in the rescue cell and subsequent assembly of viral particles at the cell's surface, newly generated rdSeV vectors are released via budding from the cell and may be used for another round of infection of helper cells.

The helper cells (HPs) are used for amplifying the SeV vectors initially assembled in the rescue cell and are typically derived from mammalian cells, such as Vero cells or HEK-293 cells. These helper cells express the P protein and, optionally the N and/or L protein. The corresponding P, N and L genes may be integrated in the helper cells' genome or present in one or more expression plasmids. An exemplary suitable cell line is a cell line derived from HEK-293 cells, which constitutively express the SeV P protein (Willenbrink et al., J. Virol. 68:8413-8417, 1994). According to the present invention, the helper cells are preferably genetically modified to express the viral P and N proteins but not the viral L protein, since this P/N co-expression was surprisingly found to result in the highest virus production rates.

In a third aspect, the present invention provides a method for producing the genome replication-deficient Sendai virus (SeV) vector of the present invention, comprising the step of:
(i) culturing a host cell of the present invention, and
(ii) collecting the genome replication-deficient SeV vector from the cell culture.

Methods for producing genome replication-deficient SeV vectors are known in the art and described in, for example, Wiegand et al., J. Virol. 81:13835-13844 (2007), Bossow et al., Open Virol. J. 6:73-81 (2012), and WO 2006/084746 A1. In the culturing step (i), the host cell is cultured in a suitable culture medium under conditions which permit genome replication and transcription so that the genome replication-deficient SeV of the present invention is formed. The medium used to culture the cells may be any conventional medium suitable for growing the host cells, such as DMEM (Invitrogen) supplemented with 10% heat-inactivated FCS. The host cell may be a rescue cell or a helper cell as defined above. In the collecting step (ii), the formed SeV vector of the present invention is recovered by methods known in the art.

In accordance with a preferred embodiment, the method for producing the genome replication-deficient Sendai virus (SeV) vector of the present invention comprises the following steps:
(a) introducing a DNA molecule into a first host cell, wherein the DNA molecule encodes the nucleic acid of the genome replication-deficient Sendai virus (SeV) vector of the present invention, or a complement thereof,
(b) culturing the first host cell to generate the genome replication-deficient SeV vector,
(c) collecting the genome replication-deficient SeV vector from the first cell culture,
(d) infecting a second host cell with the genome replication-deficient SeV vector obtained in step (c),
(e) culturing the second host cell to amplify the genome replication-deficient SeV vector,
(f) collecting the genome replication-deficient SeV vector from the second cell culture.

The first host cell is preferably a rescue cell (virus generating cell) as described above, and the second host cell is preferably a helper cell (amplification cell) as described above. The introduction of the DNA molecule into the first host cell in step (a) can be carried out by transfection methods known in the art. The culturing and collecting steps may be carried out as defined above.

In a fourth aspect, the present invention relates to a vaccine comprising the genome replication-deficient Sendai virus (SeV) vector of the present invention and one or more pharmaceutically acceptable carriers.

The term "vaccine", as used herein, refers to an agent or composition containing an active component effective to induce a therapeutic degree of immunity in a subject against a certain pathogen or disease. The vaccine of the present invention is a "semi-live" vaccine, which refers to a vaccine that is not a live vaccine since it is replication-deficient, but is also not an inactivated (or killed) vaccine since it is still capable of primary transcription and gene expression. The semi-live vaccine of the present invention is exceptionally effective (like "live vaccines") and yet particularly safe (like "dead vaccines").

In the context of the present application, the dosage form of the vaccine of the present invention is not particularly limited and may be a solution, suspension, lyophilized material or any other form suitable for the intended use. For example, the vaccine may be in the form of a parenteral formulation, such as an aqueous or non-aqueous solution or dispersion for injection or infusion, or a formulation suited for topical or mucosal administration.

The vaccine generally includes an effective amount of the rdSeV of the present invention. Within the present invention, the term "effective amount" refers to the amount of a compound sufficient to effect beneficial or desired therapeutic results. A therapeutically effective amount can be administered in one or more administrations, applications or dosages and is not intended to be limited to a particular formulation or administration route.

Further included in the vaccine are one or more pharmaceutically acceptable carriers. The term "pharmaceutically acceptable", as used herein, refers to those compounds or substances which are, within the scope of sound medical judgment, suitable for contact with the tissues of mammals, especially humans, without excessive toxicity, irritation, allergic response and other problem complications. The term "carrier", as used herein, relates to diluents, adjuvants, excipients, vehicles or other compounds or substances needed, required or desired in a vaccine composition. Suitable carriers are especially those suited for parenteral, mucosal or topical administration, including sterile aqueous and non-aqueous solutions or dispersions for injection and infusion, as discussed in Remington: The Science and Practice of Pharmacy, 20th edition (2000).

In particular, the vaccine may comprise one or more adjuvants. The term "adjuvant", as used herein, refers to an agent that enhances the immunogenicity of an antigen but is not necessarily immunogenic. Suitable adjuvants include, but are not limited to, 1018 ISS, aluminum salts, Amplivax®, AS 15, BCG, CP-870,893, CpG7909, CyaA, dSLIM, flagellin or TLR5 ligands derived from flagellin, FLT3 ligand, GM-CSF, IC30, IC31, Imiquimod (ALDARA®), resiquimod, ImuFact IMP321, interleukins such as IL-2, IL-13 , IL-21, IFN-alpha or -beta, or pegylated derivatives thereof, IS Patch, ISS, ISCOMATRIX, ISCOMs, Juvlmmune, LipoVac, MALP-2 or natural or synthetic derivatives thereof, MF59, monophosphoryl lipid A, Montanide IMS 1312, Montanide ISA 206, Montanide ISA 50V, Montanide ISA-51, water- in-oil and oil-in-water emulsions, OK-432, OM-174, OM-197-MP-EC, ONTAK, and OspA.

In addition, the vaccine may include one or more additional active substances that are co-administered with the rdSeV vector of the present invention. In addition, the pharmaceutical composition may contain additional pharmaceutical acceptable substances, for example pharmaceutical acceptable excipients such as solubilizing agents, surfactants, tonicity modifiers and the like.

In a fifth aspect, the present invention relates to a genome replication-deficient Sendai virus (SeV) vector of the present invention for use in the treatment of RSV infection or RSV infection-related diseases in a mammal.

The term "treatment", as used herein, is intended to refer to both therapeutic treatment and prophylactic treatment (or prevention) of a disease. In accordance with the present invention, "treatment" preferably means prophylactic treatment or prevention. A "treatment" within the meaning of the present invention generally involves the administration of an effective amount of the rdSeV vector of the present invention. Preferably, the rdSeV of the present invention is administered in the form of a vaccine composition as described herein.

The mammal to be treated is preferably a human subject. Particularly important target groups are human infants and children, in particular a human infant born prematurely or a human infant at risk of hospitalization for a RSV infection. Other important target groups include elderly humans, human immunocompromised individuals, transplant recipients, especially organ transplant recipients, and individuals suffering from a chronic disease. The chronic disease may be, for example, cancer, chronic hepatitis, ischemic cardiopathy, chronic renal failure, chronic respiratory diseases (e.g., asthma, obstructive pulmonary disease (COPD), pulmonary hypertension), chronic graft-versus-host disease (GVHD), and autoimmune diseases (e.g., lupus erythematosus, ulcerative colitis, inflammatory bowel diseases (IBD), Crohn's disease).

The RSV infections include all type of respiratory tract infections associated with RSV. The RSV infection-related diseases are preferably selected from the group consisting of otitis media, bronchilitis, eosinophilia, pneumonia, asthma, and chronic obstructive pulmonary disease (COPD).

Suitable administration routes include, but are not limited to, parenteral, mucosal and topical administration. The parenteral administration may be by subcutaneous, intravenous, intraperitoneal or intramuscular injection. Mucosal administration may include administration to an airway surface, such as by droplet administration to a nasal surface or sublingual administration, or by inhalation administration of aerosolized particles to a nasal surface or the surfaces of other airway passages.

As demonstrated in the examples below, the genome replication-deficient SeV vector of the present invention effectively elicits mucosal immune responses when administered intranasally. Therefore, although the genome replication-deficient SeV vector or vaccine of the present invention may be administered via any traditional route, it is preferably administered mucosally, for example via the nasal or oral (intragastric) routes. Particularly preferred is the intranasal administration.

The administration regimen is not particularly limited and includes, for example, daily, bi-weekly, monthly, once every other month, once every third, sixth or ninth month and once-a-year or single application administration schemes. The therapeutically effective dose of the virus vector that is administered to the patient depends on the mode of application, the type of disease, the patient's weight, age, sex and state of health, and the like. Administration can be single or multiple, as required. The vaccine of the present invention may also be co-administered with antigens from other pathogens as a multivalent vaccine.

The present invention and disclosures will now be further illustrated by the following, non-limiting examples.

### EXAMPLES

In the following Examples, the genetic stability and safety, production efficiency, and immunogenicity of a replication-deficient Sendai virus vector of the present disclosure (in the following referred to as "rdSeV-F_{RSV/SeV}" vector) were evaluated. The results show that the rdSeV-F_{RSV/SeV} vector is not only safe but exhibits a substantial level of chimeric RSV/SeV gene expression sufficient to induce significant serum IgG, mucosal IgA, and cytotoxic T cell responses in a mouse model. In addition, high levels of neutralizing antibodies could be induced to RSV. These characteristics make the genome replication-deficient SeV vector of the present invention a very promising viral vector vaccine candidate against RSV infections and RSV infection-related diseases.

### Materials and Methods

The following materials and methods were used in Examples 1-5.

### Cells and viruses:

Vero (ATCC CCL-81), HEp-2 (ATCC CCL-23) and P815 cells (ATCC TIB-64) from the American Type Culture Collection (Rockville, MD, USA) were maintained in Eagle minimal essential medium or RPMI (Invitrogen, Milan, Italy) supplemented with 5% heat-inactivated foetal bovine serum (FBS; Invitrogen), 100 µg/ml streptomycin and 100 U/ml penicillin. The helper cell line "P-HC" ("amplification cells") is derived from Vero cells expressing SeV phosphoprotein (protein P) (Wiegand et al., J. Virol. 81:13835-13844, 2007), and the helper cell line "VPN" is derived from Vero cells expressing the plasmid-encoded SeV phosphoprotein (protein P) and nucleoprotein (protein N). BSR-T7 cells ("rescue cells") (Buchholz et al., J. Virol. 73:251-259, 1999) were kindly provided by Klaus-K. Conzelmann (Munich). RSV type A (Long strain, ATCC VR-26) was cultured on HEp-2 cells at 37 °C. All vaccine candidates (rdSeV-F_{RSV/SeV}, rdSeV-F_{RSV/SeV}-ΔCT, rdSeV-sF_{RSV}) based on recombinant SeV vectors derived from Sendai virus strain D52 (ATCC VR-105) were cultured at 33 °C.

### Genomic vector design:

For the construction of a virus vector of the present disclosure, plasmids containing the cDNA of the RSV or SeV F gene, respectively, were used as templates for the construction of a chimeric RSV/SeV F ORF by an overlapping PCR technique (Horton et al., Gene 77:61-68, 1989). Via specific primer design, nonoverlapping regions at the 3'- and 5'-ends containing specific sequences for the restriction enzymes *Sal*I and *Xho*I, were introduced. The sequence-verified chimeric ORF was inserted into a subgenomic plasmid construct, comprising the Sendai virus genome from the *San*DI restriction site within the P gene of the wild-type genome (genomic nucleotide position 2714) until the *San*DI restriction site within the L gene (genomic nucleotide position 9131). This genomic fragment was modified in a way that the F ORF was flanked by the restriction sites for *Sal*I and *Xho*I*.* After insertion of the chimeric F ORF into the intermediate cloning vector the full length genome of rdSeV-F_{RSV/SeV} was created via transfer of the *San*DI fragment from the cloning vector into the previously prepared, full length construct of rdSeV. The resulting recombinant SeV genome, following the "rule of six" (Calain et al., J. Virol. 67:4822-4830, 1993), was designated "rdSeV-F_{RSV/SeV}" (replication- deficient SeV encoding a chimeric RSV/SeV F protein), and was confirmed by restriction analysis and sequencing.

The rdSeV-sF_{RSV} vector expressing a soluble RSV F protein was generated by transferring the subgenomic EcoRI fragment from the recombinant Sendai vector encoding the soluble form of the RSV F protein as additional transgene between the P and the M gene, as described by Voges et al. (Voges et al., Cell. Immunol. 247:85-94, 2007), into a replication-deficient Sendai vector as described in WO 2006/084746 A1. The resulting recombinant SeV genome, following the "rule of six" (Calain et al., J. Virol. 67:4822-4830, 1993), was designated "rdSeV-sF_{RSV}" (replication-deficient SeV vector expressing RSV soluble F protein), and was confirmed by restriction analysis and sequencing.

### Virus rescue, propagation and titration:

Recombinant viruses were recovered from transfected BSR-T7 cells as described in Wiegand et al., J. Virol. 81:13835-13844, 2007 with slight modifications. FuGENE6 (Roche) was used as transfection reagent at 2.0 µl/µg DNA. Replication-deficient SeV virus was harvested from the supernatant and amplified in a helper cell line stably expressing the SeV P protein ("P-HC"). This P-HC line was used in all experiments, except for the experiments in relation to virus production efficiency (see FIG. 4), where the vaccine vector rdSeV-F_{RSV/SeV} was produced in a VPN helper cell line stably expressing the Sendai virus P and N proteins (Wiegand et al., J. Virol. 81:13835-13844, 2007). Viruses were titrated as previously described (Wiegand et al., J. Virol. 81:13835-13844, 2007) and titers were given as cell infectious units per millilitre (ciu/ml) (equivalent to fluorescent plaque forming units). The integrity of the different SeV vectors was confirmed by RT-PCR and sequencing.

### Western blot analysis:

Extracts from Vero cells, mock infected or infected with PIV3, RSV or rdPIRV, were collected and separated by SDS-PAGE. After blotting on a nitrocellulose membrane proteins were detected with mouse monoclonal antibodies against PIV3 HN and F proteins (Chemicon, Milan, Italy) and a goat anti-RSV antibody (Meridian Life Science, Saco, ME).

### Animals and immunization

Each experiment was repeated at least three times to ensure reproducibility of results. Female BALB/c mice at 4-6 weeks of age were purchased from Charles River Laboratories (Milan, Italy). Four groups of 6 mice were immunized intranasally or intramuscularly. Mice received either 1.2 x 10⁶ pfu/dose of rdSeV-F_{RSV/SeV} or 1.2 x 10⁷ pfu/dose of rdSeV-F_{RSV/SeV}-sF or 1.0 x 10⁵ pfu/dose of RSV live or phosphate buffer (PBS) within a volume of 20 µl. The intranasal administration was split into 10 µl per nostril. Each group was inoculated three times, namely at day 0 (prime), day 21 (first boost) and day 42 (second boost). At day 56, mice were sacrificed for collection of bronchoalveolar lavages (BAL), nasal washes (NW), serum and spleen cells as described elsewhere (Cusi et al., Vaccine 20:3436-3442, 2000). All animal experiments complied with all relevant institutional policies, according to European Parliament directive 2010/63/EU (The European Parliament and the council of the European Union, Directive 2010/63/EU on the protection of animals used for scientific purposes, Official Journal of the European Union 2010 (22 September), L276:33-79).

### ELISA:

IgG and IgA antibodies were measured by enzyme-linked immunosorbent assay. For the determination of virus-specific IgG and IgA antibodies, purified virions (1 µg/ml) of inactivated human RSV type A (Experteam, Venice, Italy) were used as antigen, as previously described (Cusi et al., Vaccine 20:3436-3442, 2000). Results were expressed as the mean ±SD of two determinations from three different experiments. Differences were determined by the Mann-Whitney Rank Sum test.

### Neutralization assay:

Virus neutralization assay was carried out on Hep-2 cells for RSV in a 96-well microplate. Briefly, serial two-fold dilutions of immunized mice serum were added to an equal volume of RSV containing 100 TCID₅₀ in 50 µl and incubated for 90 min at 37°C, followed by the addition of 5 x 10³ cells to each well. The presence of a cytopathic effect (CPE) was examined four days later. The antibody titer was evaluated as the highest dilution that resulted in a 50% reduction of CPE. The assay was performed twice. An antibody titer of < 4 was considered negative.

### Cytokine and cytotoxicity assays:

Splenocytes drawn from immunized mice and lymphocytes were collected by Fycoll-Hypaque (Pharmacia Biotech, Uppsala, Sweden) gradient. 2 x 10⁵ of unfractionated cells in RPMI1640 plus 10% FCS were cultured in a total volume of 200 µl with 10 µg/ml of purified inactivated virus or 5 µg/ml Concanavalin A (Sigma, Milan, Italy). Control wells received cell suspension only. After 48 h in culture, cell-free supernatants were harvested and analyzed for the presence of IFN-gamma. Samples were stored at -80°C. The assay was performed as previously described (Cusi et al., Vaccine 20:3436-3442, 2002).

To assess cytotoxicity, splenocytes (1 x 10⁶ cells/ml) from each mouse of the same group were pooled and cultured for six days in RPMI (5% FCS, 100 U/ml streptomycin, 100 mg/ml penicillin). Subsequently, the cells were incubated with 50 units/ml recombinant IL-2 (Peprotech, Rocky Hill, NJ/USA) in 24-well plates for two days, followed by stimulation with inactivated PIV3 or RSV (10 µg/ml) for three more days. On day 5, target cells, represented by PIV3 or RSV (MOI 5) infected P815 cells, were labelled with 100 µCi of Na₂Cr⁵¹O₄ (Amersham, Aylesbury, UK) for 60 minutes at room temperature. Target cells (0.5 x 10⁴) in 100 µl of complete medium were added to each well in 96-well flat-bottomed assay plates (Corning Costar Corp., USA). The splenocytes were then suspended in 100 µl of RPMI medium (Invitrogen, Milan, Italy) and added to the target cells at ratios of 80:1, 40:1 and 20:1.

The plates were incubated at 37°C for 6 hours and the supernatants were harvested for γ-counting with harvester frames (Skatron Inc., Sterling, VA/USA). Uninfected P815 target cells were used as a control, while MHC class I CTL cytotoxic restriction was tested against P815 infected cells and exposed to anti-MHC class I mAb (H-2K^{d}/H-2D^{d}) (Pharmingen, Milan, Italy) before performing the assay. Measurements were done in triplicates and standard deviations were calculated. The experiments were repeated at least three times.

The percentage of specific lysis was calculated as follows: 100 x [(experimental release - spontaneous release) / (maximal release - spontaneous release)]. Spontaneous release was determined from the wells to which 100 µl of complete medium had been added, instead of effector cells. Total releasable radioactivity was obtained after treating the target cells with 2.5% TritonX-100.

### Statistical analysis

Data were normally distributed and analyzed by a non-parametric Mann-Whitney test using StatView statistical software (Abacus Concepts, Berkeley, Canada). Probability (P) values of <0.05 were considered statistically significant.

### Example 1

### Generation of an inventive replication-deficient SeV vector

Using reverse genetic techniques, a SeV vaccine vector against human RSV, named "rdSeV-F_{RSV/SeV}" (replication-deficient SeV vector expressing chimeric RSV/SeV F protein), was constructed. The SeV F ORF, except for the cytoplasmic and transmembrane domains, was replaced by its RSV counterpart to give a chimeric RSV/SeV F surface protein (FIG. 1). In addition, in order to develop a safe vaccine vector, the SeV backbone was modified in the phosphoprotein (P) gene by deleting the N-terminal 76 amino acids (PΔ2-77). As shown previously, a SeV vector with the deletion PΔ2-77 is unable to synthesize new genomic templates in non-helper cell lines, but it still capable of primary transcription and gene expression (Bossow et al., Open Virol. J. 6:73-81, 2012). The rdSeV-F_{RSV/SeV} could be rescued successfully from cDNA and amplified using the helper cell line "P-HC".

### Example 2

### Genetic stability of replication-deficient SeV vectors

In this example, the genetic stability of genome replication-deficient SeV vectors was evaluated using a specific replication-deficient SeV construct referred to as "rdPIRV" (replication-deficient PIV3/RSV SeV vector). Although this construct is not within the scope of the appended claims, the results obtained for this construct with regard to stability are also considered valid for the genome replication-deficient SeV vector of the present invention.

The rdPIRV vector is genetically engineered to express a soluble RSV F protein as well as chimeric RSV/SeV F and HN surface proteins using techniques described above and/or known in the art. In brief, the RSV F ectodomain coding sequence was inserted as an additional transcription unit being expressed as soluble protein (sF) as successfully employed previously (Voges et al., Cell. Immunol. 247:85-94, 2007). The SeV F and HN ORFs were replaced, except for the cytoplasmic and transmembrane domains, by their PIV3 counterparts. Furthermore, in order to develop a safe vaccine vector, the SeV backbone was modified in the phosphoprotein (P) gene by deleting the N-terminal 76 amino acids (PΔ2-77).

The rdPIRV could be rescued successfully from cDNA and amplified using a helper cell line. This vector was unable to synthesize new genomic templates in non-helper cell lines, but it was still capable of primary transcription and gene expression, as demonstrated by Western Blot analysis of PIV3 F and HN and RSV sF protein expression (data not shown). Further, sequence analyses after ten consecutive passages revealed no mutations.

These results confirm the structural integrity and sequence stability of the replication-deficient SeV/PΔ2-77 vaccine vector and, thus, of the replication-deficient SeV vector of the present invention.

### Example 3

### Safety of replication-deficient SeV vectors

In addition, studies regarding the safety of replication-deficient SeV vectors, in particular on replication-deficiency and biodistribution to different tissues *in vivo,* were performed with the rdPIRV vector described in Example 2. Again, the results obtained for the rdPIRV vector with regard to safety are considered to equally apply to the genome replication-deficient SeV vector of the present invention.

Two groups of BALB/C mice (n=4) were inoculated intranasally (i.n.) with 1 x 10⁵ ciu of rdPIRV or a modified replication-competent SeV (SeV-E wt) expressing the EGFP (Enhanced Green Fluorescent Protein) to facilitate its detection. After three days, mice were sacrificed and lungs and blood samples were collected. Virus present in tissue homogenates and blood was quantified by counting EGFP-positive foci on cell culture (detection limit: 20 ciu per lung, per spleen or per 500 µl blood).

No viral particles of rdPIRV could be detected in any animal tissue examined. Only when SeV-E wt was used, viral particles could be detected in the lungs (up to 3.2 x 10⁴ ciu per lung), but not in blood (data not shown). In addition, lung homogenates drawn from rdPIRV-immunized mice were overlayed onto Vero cells to verify the absence of any replicating recombinant SeV. No virus could be detected, confirming that this vaccine vector was replication-deficient *in vivo* (data not shown). No animal developed any signs of pain or weight loss.

Taken together, these data demonstrate that: (i) deletion of amino acids 2-77 in the P gene disables the vector from producing progeny genomes *in vivo;* (ii) replication competent SeV spreading is limited to the respiratory tract. These results also apply to the replication-deficient SeV vector of the present invention, which is therefore considered particularly safe for administration to humans.

### Example 4

### Production efficiency

Production efficiency of commercial vaccines has a huge impact on the market potential of such products. Therefore, production efficiency of the genome replication-deficient SeV vector of the present disclosure (rdSeV-F_{RSV/SeV} vector) was assessed and compared with that of the variant of the present invention, rdSeV-F_{RSV/SeV}-ΔCT lacking the cytoplasmic domain.

In a first study, VPN helper cells stably transfected with the genes coding for the SeV P and N proteins were infected with the inventive rdSeV-F_{RSV/SeV} vector. Different passages of the vector (P1, P2, P3) were analyzed. For passage P1 and P2 even two separate production runs were performed (P1-1, P1-2, P2-1, P2-2). The samples taken at different time points (e.g., at day 8-11 ("d8-11"), day 11-12 ("d11-12"), and so forth) from the cell culture supernatants were analyzed for their vector titers.

As can be seen from FIG. 4, the virus titers are remarkably high at all passaging levels and production runs, and significantly increase during the later passages (P2 and P3). Overall, these results demonstrate unexpectedly high production efficiency due to the presence of two surface proteins (F and HN) from two different viruses at the same time. This finding was surprising since a strong interference during the processes of attachment fusion and budding was expected.

In a second study, the production efficiency of rdSeV-F_{RSV/SeV} was compared with a variant thereof coding for a chimeric F protein lacking the cytoplasmic tail ("rdSeV-F_{RSV/SeV}-ΔCT") (see FIG. 2). This variant was spontaneously generated during sequential passaging of rdSeV-F_{RSV/SeV} on the helper cell line "P-HC". Subsequent sequence analysis of the produced vector particles revealed that a nonsense mutation in the K551 (Lys-551) codon of the F gene resulted in a premature stop codon. As a consequence, only the first two amino acids of the SeV F cytoplasmic domain (i.e. amino acids 524 and 525) are retained in this variant, which therefore (essentially) lacks its cytoplasmic tail.

During subsequent passaging of the spontaneously generated variant without cytoplasmic tail in cell culture, it was observed that the ratio of the deletion variant to non-mutated virus (rdSeV-F_{RSV/SeV}) increased. Based on this unexpected observation, it was subsequently confirmed by means of comparative production rounds in cell cultures of non-mutated virus (i.e. rdSeV-F_{RSV/SeV}) and mutated variant (i.e. rdSeV-F_{RSV/SeV}-ΔCT) that the mutant virus could be amplified to a significantly higher titer. In brief, cells were infected with the same MOI of 0.1 and cultured for five days. At different times points, i.e. at day 3 ("d2-3"), day 4 ("d3-4"), and day 5 ("d4-5"), the vector titers of cell culture supernatants were determined.

As can be seen from FIG. 5, as early as at day 3 the titer of rdSeV-F_{RSV/SeV}-ΔCT was 5-fold higher than that of rdSeV-F_{RSV/SeV}. At day 4 and day 5, respectively, the titer of rdSeV-F_{RSV/SeV}-ΔCT was even more than 10-fold higher than that of rdSeV-F_{RSV/SeV}. This finding was altogether unexpected since the prior art teaches that the cytoplasmic tail of the SeV F protein plays a critical role in virus assembly (see Stone, R. and Takimoto, T., PLoS ONE 8(4): e61281. doi:10.1371/ journal.pone.0061281, 2013). Thus, if anything, the skilled person would have expected to obtain decreased production efficiency. However, the deletion mutant rdSeV-F_{RSV/SeV}-ΔCT was surprisingly found to exhibit excellent production efficiency, even much better than that of rdSeV-F_{RSV/SeV} expressing the full-length chimeric RSV/SeV F protein.

The above results show that the rdSeV vector of the present invention can be produced in a highly efficient manner. In addition, deletion of the cytoplasmic domain of the chimeric RSV/SeV protein results in an even higher production capacity.

### Example 5

### Immunogenicity of the replication-deficient SeV vector

### Specific antibody response (IgG and IgA):

To evaluate the capability of rdSeV-F_{RSV/SeV} to induce specific immune responses, its immunogenicity was compared with (i) the replication-deficient SeV vector expressing soluble RSF F protein ("rdSeV-sF_{RSV}") shown in FIG. 3, (ii) the Long strain (ATCC VR-26) of RSV ("RSV live") as positive control, and (iii) polyphosphate-buffered saline (PBS) as negative control. Furthermore, two different routes of administration were evaluated, i.e. intranasal (i.n.) and intramuscular (i.m.). The same dose of each vector was applied via both administration routes.

Mice were i.n. and i.m. infected with rdSeV-sF_{RSV}, rdSeV-F_{RSV/SeV}, RSV live and PBS as described above. As shown in FIG. 6, mice immunized with rdSeV-F_{RSV/SeV} developed a much stronger IgG antibody response against RSV than rdSeV-sF_{RSV} upon both i.n. and i.m. administration. Thus, encoding RSV F antigen as structural protein appears to be advantageous compared to encoding the RSV F antigen solely as additional transgene leading to its soluble protein conformation.

It was further observed that the intranasal administration of rdSeV-F_{RSV/SeV} resulted in a significantly stronger induction of mucosal antibodies (IgA) in nasal washes (see FIG. 7) and in bronchoalveolar lavages (see FIG. 8). This strong induction of IgA antibodies by rdSeV-F_{RSV/SeV} is a very advantageous property of the SeV vector of the present invention because it represents a strong first line of defense upon the encounter of respiratory pathogens taken up via the respiratory tract like RSV.

The rdSeV-F_{RSV/SeV} was also able to induce high levels of neutralizing antibodies against RSV. In contrast, as can be seen from FIG. 9, the levels of neutralizing antibodies induced by rdSeV-sF_{RSV} after i.n. as well as i.m. administration were markedly lower. This shows that rdSeV-F_{RSV/SeV} is capable of inducing a good neutralizing immune response to RSV, which is considered important in eliciting a potent immune response.

### Specific T cell responses (IFN-gamma expression and CTL response):

In order to verify whether the replication-deficient SeV vector of the present invention is able to induce RSV-specific T cell responses, the induction of IFN-gamma was evaluated. As it can be seen from FIG. 10, a relevant level of IFN-gamma was produced by splenocytes, following *in vitro* re-stimulation with inactivated RSV, indicating a robust T helper 1 (Th1) response, which is regarded as being indicative for the induction of specific cytotoxic T cells. In particular, the production of IFN-gamma was higher in mice immunized i.n. with rdSeV-F_{RSV/SeV} than in those receiving rdSeV-sF_{RSV}.

Moreover, it was investigated whether i.n. or i.m. immunization with rdSeV-F_{RSV/SeV} is capable of eliciting a cytotoxic T cell response against RSV. A specific cytolysis was revealed by the splenocytes of immunized mice versus target cells infected with RSV (see FIG. 11 and FIG. 12). Mice inoculated with rdSeV-F_{RSV/SeV} showed a stronger CTL response against RSV than rdSeV-sF_{RSV}, indicating that the vaccine vector encoding the RSV F protein as structural protein (i.e. the chimeric RSV/SeV surface protein F) was able to efficiently stimulate a specific cell-mediated immune response.

In conclusion, the results presented in the above examples show that the rdSeV vector of the present invention is able to highly efficiently stimulate different immune responses. The extent of the induced immune responses could not be expected given the fact that the rdSeV of the present invention is genome replication-deficient and, thus, there exists much less RNA templates present during infection compared to wild-type SeV. In addition, the quality of the induced immune response indicates that different arms of the adaptive immune system can be stimulated, including serum antibodies, mucosal antibodies and specific cellular T cells.

Furthermore, based on its natural route of infection, the rdSeV vector of the present invention is perfectly suited for mucosal application, enabling an IgA response as a strong first line of defense against RSV, as shown above. A strong IgA response is, together with cellular responses, of paramount importance for the efficacy of a vaccine against respiratory pathogens such as RSV.

Moreover, the rdSeV vector of the present invention can be produced in an unexpectedly efficient manner. A high production efficiency is a highly important and desirable feature with regard to commercialization as a vaccine. Thus, the rdSeV vector of the present invention is a very promising vaccine candidate against RSV.

## Claims

1. A genome replication-deficient Sendai virus (SeV) vector comprising a nucleic acid that is modified in the phosphoprotein (P) gene to encode a mutant P protein lacking amino acids 2-77, wherein the nucleic acid further encodes a chimeric F protein comprising a respiratory syncytial virus (RSV) F ectodomain and a SeV F transmembrane domain, wherein the chimeric F protein essentially lacks a SeV F cytoplasmic domain.

2. The genome replication-deficient SeV vector of claim 1, wherein the RSV ectodomain corresponds to amino acids 1-524 of a RSV F protein and / or the SeV transmembrane domain corresponds to amino acids 500-523 of a SeV F protein.

3. The genome replication-deficient SeV vector of claims 1 to 2, wherein the nucleic acid additionally encodes a soluble RSV F protein.

4. The genome replication-deficient SeV vector of claim 3, wherein the soluble RSV F protein is the ectodomain of a RSV F protein.

5. The genome replication-deficient SeV vector of claims 1 to 2, wherein the nucleic acid does not additionally encode a soluble RSV F protein.

6. A host cell comprising a genome replication-deficient Sendai virus (SeV) vector according to any one of claims 1 to 5, the nucleic acid of the genome replication-deficient SeV vector according to any one of claims 1 to 5 or a complement thereof, and / or a DNA molecule encoding the nucleic acid of the genome replication-deficient SeV vector according to any one of claims 1 to 5 or encoding a complement of the nucleic acid.

7. A method for producing the genome replication-deficient Sendai virus (SeV) vector according to any one of claims 1 to 5, comprising:
(i) culturing a host cell according to claim 6, and
(ii) collecting the genome replication-deficient SeV vector from the cell culture.

8. A vaccine comprising the genome replication-deficient Sendai virus (SeV) vector according to any one of claims 1 to 5 and one or more pharmaceutically acceptable carriers.

9. The vaccine of claim 8, further comprising an adjuvant.

10. A genome replication-deficient Sendai virus (SeV) vector of any one of claims 1 to 5 for use in the treatment of RSV infections or RSV infection-related diseases in a mammal.

11. The genome replication-deficient SeV vector for use according to claim 10, wherein the mammal is a human subject.

12. The genome replication-deficient SeV vector for use according to claim 10 or 11, wherein the human subject is a human infant or child, including a human infant born prematurely or a human infant at risk of hospitalization for a RSV infection, an elderly human, a human immunocompromised individual, a transplant recipient, or an individual suffering from a chronic disease.

13. The genome replication-deficient SeV vector for use according to any one of claims 10 to 12, wherein the vaccine is administered parenterally, topically or mucosally.

14. The genome replication-deficient SeV vector for use according to claim 13, wherein the parenteral administration is by subcutaneous, intravenous, intraperitoneal or intramuscular injection.

## Patentansprüche

1. Genomreplikations-defizienter Sendai-Virus(SeV)-Vektor, umfassend eine Nukleinsäure, die in dem Phosphoprotein(P)-Gen modifiziert ist, um ein mutiertes P-Protein zu kodieren, dem die Aminosäuren 2-77 fehlen, wobei die Nukleinsäure ferner ein chimäres F-Protein kodiert, das eine F-Ektodomäne des respiratorischen Synzytialvirus (RSV) und eine SeV-F-Transmembrandomäne umfasst, wobei dem chimären F-Protein im Wesentlichen eine zytoplasmatische SeV-F-Domäne fehlt.

2. Genomreplikations-defizienter SeV-Vektor gemäß Anspruch 1, wobei die RSV-Ektodomäne den Aminosäuren 1-524 eines RSV-F-Proteins entspricht und/oder die SeV-Transmembrandomäne den Aminosäuren 500-523 eines SeV-F-Proteins entspricht.

3. Genomreplikations-defizienter SeV-Vektor gemäß Ansprüchen 1 bis 2, wobei die Nukleinsäure zusätzlich ein lösliches RSV-F-Protein kodiert.

4. Genomreplikations-defizienter SeV-Vektor gemäß Anspruch 3, wobei das lösliche RSV-F-Protein die Ektodomäne eines RSV-F-Proteins ist.

5. Genomreplikations-defizienter SeV-Vektor gemäß Ansprüchen 1 bis 2, wobei die Nukleinsäure nicht zusätzlich ein lösliches RSV-F-Protein kodiert.

6. Wirtszelle, umfassend einen genomreplikations-defizienten Sendai-Virus(SeV)-Vektor gemäß einem der Ansprüche 1 bis 5, die Nukleinsäure des genomreplikations-defizienten SeV-Vektors gemäß einem der Ansprüche 1 bis 5 oder ein Komplement davon und/oder ein DNA-Molekül, das die Nukleinsäure des genomreplikations-defizienten SeV-Vektors gemäß einem der Ansprüche 1 bis 5 kodiert oder ein Komplement der Nukleinsäure kodiert.

7. Verfahren zum Herstellen des genomreplikations-defizienten Sendai-Virus(SeV)-Vektors gemäß einem der Ansprüche 1 bis 5, umfassend:
(i) Kultivieren einer Wirtszelle gemäß Anspruch 6 und
(ii) Sammeln des genomreplikations-defizienten SeV-Vektors aus der Zellkultur.

8. Impfstoff, umfassend den genomreplikations-defizienten Sendai-Virus(SeV)-Vektor gemäß einem der Ansprüche 1 bis 5 und einen oder mehrere pharmazeutisch verträgliche Träger.

9. Impfstoff gemäß Anspruch 8, ferner umfassend einen Hilfsstoff.

10. Genomreplikations-defizienter Sendai-Virus(SeV)-Vektor gemäß einem der Ansprüche 1 bis 5 für die Verwendung bei der Behandlung von RSV-Infektionen oder mit RSV-Infektion verbundenen Erkrankungen bei einem Säuger.

11. Genomreplikations-defizienter SeV-Vektor für die Verwendung gemäß Anspruch 10, wobei der Säuger ein menschliches Subjekt ist.

12. Genomreplikations-defizienter SeV-Vektor für die Verwendung gemäß Anspruch 10 oder 11, wobei das menschliche Subjekt ein menschlicher Säugling oder menschliches Kind ist, einschließlich eines frühgeborenen menschlichen Säuglings oder eines menschlichen Säuglings unter dem Risiko eines Krankenhausaufenthalts aufgrund einer RSV-Infektion, eines älteren Menschen, eines menschlichen immungeschwächten Individuums, eines Transplantatempfängers und eines Individuums, das an einer chronischen Erkrankung leidet.

13. Genomreplikations-defizienter SeV-Vektor für die Verwendung gemäß einem der Ansprüche 10 bis 12, wobei der Impfstoff parenteral, topisch oder mukosal verabreicht wird.

14. Genomreplikations-defizienter SeV-Vektor für die Verwendung gemäß Anspruch 13, wobei die parenterale Verabreichung durch subkutane, intravenöse, intraperitoneale oder intramuskuläre Injektion erfolgt.

## Revendications

1. Vecteur de virus Sendaï (SeV) à génome déficient pour la réplication comprenant un acide nucléique qui est modifié, dans le gène d'une phosphoprotéine (P), pour coder pour une protéine P mutante dépourvue des acides aminés 2 à 77, dans lequel l'acide nucléique code en outre pour une protéine F chimère qui comprend un ecto-domaine de F du virus respiratoire syncytial (VRS) et un domaine transmembranaire de F du SeV, dans lequel la protéine F chimère est essentiellement dépourvue d'un domaine cytoplasmique de F du SeV.

2. Vecteur de SeV à génome déficient pour la réplication selon la revendication 1, dans lequel l'ecto-domaine du VRS correspond aux acides aminés 1 à 524 d'une protéine F du VRS et/ou le domaine transmembranaire du SeV correspond aux acides aminés 500 à 523 d'une protéine F du SeV.

3. Vecteur de SeV à génome déficient pour la réplication selon les revendications 1 à 2, dans lequel l'acide nucléique code, en plus, pour une protéine F soluble du VRS.

4. Vecteur de SeV à génome déficient pour la réplication selon la revendication 3, dans lequel la protéine F soluble du VRS est l'ecto-domaine d'une protéine F du VRS.

5. Vecteur de SeV à génome déficient pour la réplication selon les revendications 1 à 2, dans lequel l'acide nucléique ne code pas, en plus, pour une protéine F soluble du VRS.

6. Cellule hôte comprenant un vecteur de virus Sendaï (SeV) à génome déficient pour la réplication, selon l'une quelconque des revendications 1 à 5, l'acide nucléique du vecteur de SeV à génome déficient pour la réplication, selon l'une quelconque des revendications 1 à 5, ou un complément de ceux-ci, et/ou une molécule d'ADN codant pour l'acide nucléique du vecteur de SeV à génome déficient pour la réplication, selon l'une quelconque des revendications 1 à 5, ou codant pour un complément de l'acide nucléique.

7. Procédé de production du vecteur de virus Sendaï (SeV) à génome déficient pour la réplication, selon l'une quelconque des revendications 1 à 5, comprenant les étapes consistant à :
(i) cultiver une cellule hôte, selon la revendication 6, et
(ii) recueillir le vecteur de SeV à génome déficient pour la réplication à partir de la culture cellulaire.

8. Vaccin comprenant le vecteur de virus Sendaï (SeV) à génome déficient pour la réplication, selon l'une quelconque des revendications 1 à 5, et un ou plusieurs transporteur(s) acceptable(s) d'un point de vue pharmaceutique.

9. Vaccin, selon la revendication 8, comprenant en outre un adjuvant.

10. Vecteur de virus Sendaï (SeV) à génome déficient pour la réplication, selon l'une quelconque des revendications 1 à 5, destiné à être utilisé dans le traitement d'infections par VRS ou de maladies associées à une infection par VRS chez un mammifère.

11. Vecteur de SeV à génome déficient pour la réplication destiné à être utilisé selon la revendication 10, dans lequel le mammifère est un sujet humain.

12. Vecteur de SeV à génome déficient pour la réplication destiné à être utilisé selon les revendications 10 ou 11, dans lequel le sujet humain est un nourrisson ou un enfant humain, y compris un nourrisson humain né prématurément ou un enfant humain étant à risque d'être hospitalisé pour une infection par VRS, un être humain âgé, un individu humain immuno-compromis, un receveur de greffe ou un individu atteint d'une maladie chronique.

13. Vecteur de SeV à génome déficient pour la réplication destiné à être utilisé selon l'une quelconque des revendications 10 à 12, dans lequel le vaccin est administré par voie parentérale, topique ou par les muqueuses.

14. Vecteur de SeV à génome déficient pour la réplication destiné à être utilisé selon la revendication 13, dans lequel l'administration parentérale s'effectue par une injection sous-cutanée, intraveineuse, intra-péritonéale ou intramusculaire.
